# EUROPÄISCHE PATENTANMELDUNG

(11) **EP 2 181 701 A1**
(43) Veröffentlichungstag der Anmeldung: **05.05.2010**
(21) Anmeldenummer: 08018967.3
(22) Anmeldetag: 30.10.2008
(51) Int. Cl.: A61K 8/97, A61K 36/886, A61K 36/35, A61K 8/04, A61Q 19/00, A61P 17/02, A61P 17/16

(54) **Zusammensetzung zur äusseren, kosmetischen Anwendung auf einer elastizitätsarmen und extrem trockenen Haut**

(71) Anmelder: L'ESTÉTIC GmbH, 82031 Grünwald (DE)
(72) Erfinder: Löschnigg, Isabella, 5020 Salzburg (AT)
(74) Vertreter: Wibbelmann, Jobst

(57) **Zusammenfassung**

Die Erfindung betrifft eine Zusammensetzung zur äußeren, kosmetischen Anwendung auf einer durch Strahlen veränderten Haut, wobei die Zusammensetzung ein Gel ist, dessen wesentliche Bestandteile Aloe vera barbadensis und Sambucus nigra Extrakt sind.

## Beschreibung

Die vorliegende Erfindung betrifft eine Zusammensetzung zur äußeren, kosmetischen Anwendung auf einer durch Strahlen veränderten Haut (elastizitätsarm und extrem trocken). Diese Zusammensetzung ist ein Gel, dessen wesentliche Bestandteile Aloe barbadensis und Sambucus nigra (Holunder) Extrakt sind. Bei der elastizitätsarmen und extrem trockenen Haut kann es sich um eine durch Strahlen geschädigte Haut handeln, insbesondere durch α-, β- und/oder γ- Strahlen im Rahmen einer Strahlentherapie nach einer Krebserkrankung. Die Erfindung betrifft aber auch ein Kombinationspräparat von kosmetischen Zusammensetzungen zur äußeren, kosmetischen Anwendung auf einer durch Strahlen veränderten Haut, wobei die eine Zusammensetzung im Wesentlichen der Regenerierung der im Rahmen der Strahlungstherapie veränderten, trockenen und/oder stark irritierten Haut dient, und die zweite Zusammensetzung die gereizte Haut beruhigen und die Elastizität der Haut fördern soll, so dass die Haut in besonderer Weise auf einen wiederherstellenden, plastisch-chirurgischen Eingriff vorbereitet wird.

Sowohl die Zusammensetzung als auch das Kombinationspräparat eignen sich in besonderer Weise zur Pflege der Haut nach einem Mammakarzinom.

Das Mammakarzinom ist der häufigste bösartige Tumor bei der Frau. In Deutschland erkranken jährlich ca. 75000 Frauen an einem Mammakarzinom, weltweit sind es ca. 1000000. Die weibliche Brust besteht aus Drüsenläppchen, Fettgewebe und Milchgängen. Darüber hinaus verlaufen in ihr Blutgefäße, Nerven und Lymphgefäße. Unter der Brust liegen der große und der kleine Brustmuskel. Das Brustgewebe wird von Haut bedeckt, die durch die Brustwarze und den Warzenvorhof unterbrochen wird. Ein Mammakarzinom kann entlang der Lymphbahnen in die entsprechenden Lymphknoten (regionäre Metastasierun) oder entlang der Blutgefäße (hämatogene Metastasierung) in Knochen, Lunge und Gehirn metastasieren.

Das Auftreten von Brustkrebs steigt seit 1980 beständig an.

Die Therapie des Mammakarzinoms baut im Wesentlichen auf 3 Säulen auf:
1. Operation, 2. Bestrahlung und 3. Medikation (z.B. Chemotherapie, Hormontherapie oder Therapie mit Antikörpern). Die Operation des Karzinoms nimmt dabei einen zentralen Platz ein. Dabei werden derzeit etwa 2/3 der Operationen brusterhaltend durchgeführt. Im Anschluss an die brusterhaltende Operation wird regelmäßig eine Bestrahlungstherapie durchgeführt.

Bei brusterhaltenden Operationen ist die Strahlentherapie ein unverzichtbarer Bestandteil des Therapiekonzeptes. Häufig kann ohne eine zusätzliche Chemotherapie 4-6 Wochen nach der Operation mit einer Bestrahlung begonnen werden. In Kombination mit einer medikamentösen Therapie findet die Bestrahlung entweder nach deren Abschluss oder im Intervall statt. Nach einer operativen Entfernung der Brust (Mastektomie, Ablatio) wird eine Strahlentherapie nur beim Vorliegen bestimmter Kriterien erforderlich. Zuweilen ist aber auch eine alleinige Bestrahlung der Brust indiziert (beispielsweise aufgrund des hohen Alters oder schwerer Begleiterkrankungen der Patientin).

Bei einer Strahlentherapie im Rahmen der Behandlung eines Mammakarzinoms (gleich, ob es sich um eine brusterhaltende Operation oder die vollständig Entfernung der Mamma handelt) sind die durch die Strahlenbehandlung bedingten Hautveränderungen in besonderer Weise beeinträchtigend, und zwar nicht nur aus subjektiver Sicht der Patientin, sondern auch aus medizinischer Sicht mit Blick auf den Wiederaufbau der Mamma. Das strahlentherapeutisch behandelte Hautgebiet weist nämlich häufig eine beträchtlich verminderte Elastizität auf, die den plastisch-chirurgischen Wiederaufbau der Brust deutlich erschwert. Aus dermatologischer Sicht wird eine akute von einer chronischen Radiodermatitis unterschieden. Die akute Form kann mit Rötung, gefolgt von einer diffusen oder fleckigen Hyperpigmentierung, einer vorübergehenden Blockade der Talgdrüsensekretion oder einer befristeten Alopezie einhergehen. Bei größeren Strahlendosen können Rötungen, Ödem, Bläschen und späteres Nässen hinzukommen. Dazu kommt es im bestrahlten Bereich häufig zu einem dauernden Verlust der Haare, Talgdrüsen und größtenteils auch der Schweißdrüsen. Die chronische Form der Radiodermatitis kann 2-12 Jahre nach Bestrahlung auftreten. Charakteristisch ist eine bleibende, meist sklerotische Atrophie der Haut im Bestrahlungsgebiet. Es kommt zu einem Verlust der Anhangsgebilde, Störungen der Pigmentbildung sowie zur Ausbildung von Gefäßerweiterungen. Die Haut ist häufig trocken.

Der Schaden wird durch verschiedene Faktoren bestimmt (beispielsweise Gesamtdosis, Art der Strahlung, betroffene Haut, usw.). So führt eine Strahlung von bereits 1 Gy zu einer Reduktion der Koloniebildung der Keratinozyten um ein Drittel. Die Keratinozyten bilden den Hauptanteil der Epidermis, terminal differenzieren sie zu Korneozyten, die wiederum die Träger der Barrierefunktion der Haut darstellen. Das heißt, bereits die Bestrahlung mit kleinsten Dosen führt zu einer Funktionseinschränkung der Haut.

Zur Behandlung von Strahlenschäden an der Haut werden ganz allgemein Puder empfohlen. Insbesondere findet man in einem Leitfaden zur Therapie und Nachsorge des Mammakarzinoms aus dem Jahre 2005 noch die folgende Empfehlung zur Hautpflege:
*"Im Allgemeinen ist eine besondere Hautpflege mit Puder oder Salben zur Vorbeugung von Strahlenreaktionen nicht zwingend notwendig. Sollten im Verlauf der Behandlung Hautreizungen auftreten, wird eine spezielle Salbenbehandlung empfohlen. In randomisierten Studien konnte kein Unterschied bezüglich des Erythemgrades in Abhängigkeit von der Hautpflege mit Puder, Waschen ohne Seife, Waschen mit Seife, Panthenol, Theta-Creme, Aloe vera oder anderen getesteten Substanzen gezeigt werden (LOE II B).*
*Feuchte Epitheliolysen sind mit geeigneten. Wundverbänden zu behandeln (z.B. Algenat, Hydrocolloid, Polyfoam etc.). Farbstoffhaltige Lösungen (Bril lantgrün, Gentiana violett etc.) und mit Öl oder mit Antibiotika beschichtete Gaze (Oleotüll etc.) haben sich in der chirurgischen Wundpflege als nachteilig herausgestellt und sollten daher in der Behandlung feuchter Epitheliolysen vermieden werden."*

Es scheint also im Stand der Technik keinerlei Erkenntnis darüber vorzuliegen, wie eine durch Strahlentherapie geschädigte Haut, insbesondere wenn ein Wiederaufbau der Brust angestrebt wird, in geeigneter Weise behandelt werden kann. Jedenfalls erweist sich der von Ärzten häufig empfohlene Puder (beispielsweise einfacher Baby-Puder) als wenig geeignet. Vielmehr trocknet Puder eine durch Strahlen geschädigte Haut noch zusätzlich aus und trägt sicherlich nicht dazu bei, die Haut für einen späteren, plastisch-chirurgischen Eingriff zur Rekonstruktion der Brust vorzubereiten.

Der Erfindung liegt deshalb die Aufgabe zugrunde, ein Pflegekonzept zu entwickeln, das sich insbesondere zur äußeren, kosmetischen Anwendung auf einer im Rahmen einer Strahlentherapie veränderten Haut eignet. Dieses Pflegekonzept soll dazu dienen, die Haut bei der Regenerationsphase zu unterstützen, ihre Elastizität wiederherzustellen und für einen eventuell nachfolgenden, plastischen Eingriff vorzubereiten.

Die vorliegende Erfindung beruht auf der Erkenntnis, dass eine Kombination ausgesuchter, rein pflanzlicher Wirkstoffe das angestrebte Ziel erreichbar macht. Die Erfindung verzichtet dabei regelmäßig auf den Einsatz von demineralisiertem Wasser, von Mineralölen und von herkömmlichen Konservierungsmitteln, die eine empfindliche Haut oft zusätzlich angreifen.

Zusätzlich berücksichtigt die Erfindung auch den besonderen seelischen Ausnahmezustand, der bei Patienten mit Mammakarzinom unterstellt werden kann. Eine Krebserkrankung und die erforderliche operative und chemotherapeutische Behandlung führt nämlich zu einem seelischen Ausnahmezustand und häufig zu einem Gefühl absoluter Hilflosigkeit. Deshalb spielt es durchaus für die Therapie auch eine Rolle, dass im Rahmen der Erfindung ätherische Öle und wohlriechende Pflanzenauszüge zum Einsatz kommen.

Mit der vorliegenden Erfindung wird ein Anwendungskonzept vorgestellt, das insbesondere nach einer Bestrahlungstherapie die Regenerationsphase der Haut unterstützt, ihre Elastizität wiederherstellt, sowie ihre Dehnbarkeit für nachfolgende plastische Eingriffe fördert.

Gegenstand der Erfindung ist folglich eine Zusammensetzung zur äußeren, kosmetischen Anwendung auf einer durch Strahlen veränderten Haut (stark elastizitätsarm und extrem trocken) in Form eines Gels, das als wesentlichen Bestandteil Aloe vera barbadensis und Sambucus nigra (Holunder) Extrakt enthält. Bei der durch Strahlen veränderten Haut handelt es sich regelmäßig um die äußere Haut der Mamma nach einer Strahlentherapie (beispielsweise mit α-, β- und/oder γ- Strahlen). In einer bevorzugten Ausführungsform besteht diese Zusammensetzung zu über 50% aus Aloe barbadensis und Sambucus nigra Extrakt. Beispielsweise kann diese Zusammensetzung zu 85% Gew.-% aus Aloe barbadensis, Pentylenglycol und Sambucus nigra Extrakt bestehen. Weitere Bestandteile sind beispielsweise Saccharidisomerat, Ceratonia Siliqua, Sodium Carbomer (Acrylsäurepolymerisat), Xanthangummi, und/oder Citronensäure.

Wesentlicher Bestandteil der Zusammensetzung ist, wie bereits oben ausgeführt, ein Extrakt aus Holunderbeeren. Ein solcher Extrakt wird durch geeignete Verfahren mit Hilfe eines Extraktionsmittels aus Holunderbeeren gewonnen.

Der zweite, wesentliche Bestandteil ist Aloe vera barbadensis. Dieser Bestandteil wird aus den mindestens 5-jährigen Blättern der Aloe Pflanze gewonnen.

Diese Zusammensetzung enthält Aloe barbardensis regelmäßig in Anteilen oberhalb von 40 Gew.-%. Der Anteil von Pentylenglykol ist regelmäßig niedriger als 5 Gew.-%. Sambucus Nigra Extrakt ist in einer Menge von weniger als 40 Gew.-% vorhanden.

Gegenstand der Erfindung ist darüber hinaus ein Kombinationspräparat aus mindestens zwei getrennt aufzutragenden Kosmetikprodukten zur topischen Behandlung einer - bedingt durch Strahlentherapie - feuchtigkeits- und elastizitätsarmen Haut, wobei eine Zusammensetzung den vorstehenden Vorgaben entspricht, und die zweite Zusammensetzung der Beruhigung der gereizten Haut und/oder der Förderung der Elastizität dient. Dabei können diese Zusammensetzungen sowohl gleichzeitig als auch getrennt aufgetragen werden. Bei einer langfristigen Hautbehandlung und insbesondere bei stark betroffener Haut kann eine Behandlung zunächst mit der ersten Zusammensetzung erfolgen, wobei dann die Kombination erst zu einem späteren Zeitpunkt (zur Vorbereitung auf eine Operation) zum Einsatz kommt.

Die zweite Zusammensetzung enthält einen Aloe vera barbadensis Ölextrakt, zusätzlich Sojaöl (Glycine Soja), Prunus Amygdalus Dulcis Öl, Triticum Vulgare (Weizenextrakt) und Buxus Chinensis (Jojoba-Öl). Nach einer bevorzugten Ausführungsform enthält die zweite Zusammensetzung die vorstehenden Bestandteile mit einem Anteil von insgesamt 97 Gew.-% (bezogen auf das Gesamtgewicht der Zusammensetzung). Weitere mögliche Bestandteile sind Tocopherylacetat, Lecithin, Tocopherol, Ascorbylpalmitat, hydriertes Ölpalmenglyceridcitrat.

Diese zweite Zusammensetzung enthält regelmäßig Glycine Soja in einem Anteil von weniger als 20 Gew.-%. Prunus Amygdalus Dulcis Öl (Sweet Almond) liegt regelmäßig mit Anteilen unterhalb von 20 Gew.-% vor. Triticum Vulgare wird mit weniger als 10 Gew.-% eingesetzt, Aloe Barbadensis mit mehr als 20 Gew.-%, und Buxus Chinensis mit mehr als 20 Gew.-%. Die weiteren angegebenen Bestandteile machen dann regelmäßig weniger als 10 Gew.-% aus.

Die vorstehend erwähnten Zusammensetzungen enthalten häufig neben verschiedenen ätherischen Ölen auch noch andere Pflanzenauszüge, die in sehr geringer Konzentration eingesetzt werden.

Von besonderer Bedeutung ist, dass hier auf übliche Trägerstoffe (demineralisiertes Wasser, mineralische Öle) vollständig verzichtet werden kann. Stattdessen dienen reine Pflanzenhydrolate und pflanzliche Öle als Träger. Der Zusatz von speziellen Vitamin- und Wirkstoffkombinationen gewährleistet dann eine Haltbarkeit von 30 Monaten.

Die Herstellung der erfindungsgemäßen Zusammensetzung und Kombinationspräparate erfolgt in besonders sorgfältiger Weise, wobei insbesondere darauf geachtet wird, dass die Temperatur nicht zu hohe Werte erreicht. Beispielsweise wird darauf geachtet, dass nach und/oder bei Zugabe der Vitamine eine Temperatur von 45°C nicht überschritten wird.

Es hat sich zusätzlich gezeigt, dass die erfindungsgemäße Zusammensetzung wie auch das Kombinationspräparat als generelles Pflegeprodukt für die weibliche Brust eingesetzt werden kann. Dies zeigt sich insbesondere bei der topischen Behandlung von Narben, wie sie nach einer Operation der Mamma (brusterhaltend oder Ablatio) auftreten.

### Anwendungsbeispiel

Eine Patientin im Alter von 33 Jahren mit Brustkrebs wurde im Anschluss an eine Operation mit Chemotherapie und Bestrahlung behandelt. Die Strahlentherapie erfolgte über einen Zeitraum von 6 Wochen.

Im Anschluss an die Strahlentherapie wurde der Patientin von dem behandelnden Radiologen geraten, die bestrahlte Brust über einen Zeitraum von 6 Wochen weder zu waschen noch zu baden. Stattdessen sollte die Patientin Baby-Puder aus der Drogerie einsetzen.

Infolge der Behandlung mit Baby-Puder trocknete die bestrahlte Haut der Patientin noch weiter aus, und es zeigte sich keinerlei Verbesserung der äußerlich nahezu vollständig verbrannten Haut.

Die Patientin trug dann über einen Zeitraum von 4 Wochen mindestens 2x täglich das erfindungsgemäße Gel auf. Bereits nach 4 Wochen zeigte sich, dass die Haut regenerierte.

Zur Vorbereitung auf einen plastisch-chirurgischen Eingriff und Wiederaufbau der Brust benutzte die Patientin im Anschluss daran die zweite Zusammensetzung zusammen mit der ersten Zusammensetzung, und trug diese regelmäßig mehrfach täglich auf die betroffene Haut auf.

Zur Vorbereitung der plastisch-chirurgischen Operation ergab sich die Notwendigkeit, die Brusthaut und den Brustmuskel weiter zu dehnen. Es zeigte sich, dass die regelmäßig behandelte Haut eine sehr gute Dehnbarkeit entwickelte, so dass die Haut bereits nach einem halben Jahr soweit gedehnt war, dass eine Operation mit einem Implantat durchgeführt werden konnte.

Insgesamt zeigte sich, dass nach einer Behandlung der Haut über einen Zeitraum von 1 Jahr diese geradezu makellos war und Spuren der Strahlenbehandlung nicht mehr sichtbar waren.

## Patentansprüche

1. Zusammensetzung zur äußeren, kosmetischen anwendung auf einer durch Strahlen veränderten Haut in Form eines Gels, das als wesentliche Bestandteile Aloe vera barbadensis und Sambucus nigra (Holunder) Extrakt enthält.

2. Zusammensetzung nach Anspruch 1, wobei die Haut durch α-, β- und/oder γ-Strahlen verändert wurde.

3. Zusammensetzung nach Anspruch 1 oder 2, **dadurch gekennzeichnet, dass** die Veränderung der Haut im Rahmen einer Strahlentherapie zur Krebsbehandlung aufgetreten ist.

4. Zusammensetzung nach einem der vorstehenden Ansprüche, **dadurch gekennzeichnet, dass** der Krebs ein Mammakarzinom war.

5. Kombinationspräparat von mindestens zwei kosmetischen Zusammensetzungen zur äußeren, kosmetischen Anwendung auf einer durch Strahlen veränderten Haut, wobei die erste Zusammensetzung eine Zusammensetzung nach einem der Ansprüche 1 bis 4 ist, und die zweite Zusammensetzung der Beruhigung der gereizten Haut und/oder der Förderung der Elastizität dient, wobei die erste und zweite Zusammensetzung entweder zugleich oder im Wechsel auf die Haut aufgetragen werden.

6. Kombinationspräparat gemäß Anspruch 5, **dadurch gekennzeichnet, dass** die zweite Zusammensetzung als wesentlichen Bestandteil Aloe barbadensis enthält.

7. Kombinationspräparat nach Anspruch 5 oder 6, **dadurch gekennzeichnet, dass** die zweite Zusammensetzung ein Öl ist.

8. Kombinationspräparat nach einem der Ansprüche 5 bis 7, **dadurch gekennzeichnet, dass** die zweite Zusammensetzung der Dehnung der Haut zur Vorbereitung derselben auf einen plastisch-chirurgischen Eingriff dient.
